# EUROPEAN PATENT APPLICATION

(11) **EP 1 623 706 A2**
(43) Date of publication of application: **08.02.2006**
(21) Application number: 05019860.5
(22) Date of filing: 26.02.2002
(51) Int. Cl.: A61K 31/337, A61P 35/00

(54) **Metronomic dosing of taxanes for inhibiting tumor growth**

(30) Priority: 28.02.2001 US 271944 P
(62) Divisional of application: 02723255.2
(71) Applicant: Bristol-Myers Squibb Company, Princeton, NJ 08543-4000 (US)
(72) Inventor: Fargnoli, Joseph, Pipersville, PA 18947 (US); Rose, William C., Pipersville, PA 18947 (US); Trail, Pamela, Madison, CT 06443 (US)
(74) Representative: Vossius & Partner

(57) **Abstract**

A metronomic dosing regime for taxanes is provided which inhibits tumor growth in animals.

## Description

### RELATED APPLICATIONS

This application claims the benefit under 35 § 119(e) of U.S. Provisional Application No. 60/271,944, filed February 28, 2001.

### Background of the Invention

Traditionally, chemotherapeutic drug regimes for treatment of cancer have been designed to kill as many tumor cells as possible by treating with "maximum tolerated doses" (MTDs) of these cytotoxic agents (Hanahan et al. J. Clinical Invest. 2000 105(8):1045-1047). This MTD dosing regime is also referred to routinely as induction therapy. However, the toxic side effects associated with damage to proliferating cells in healthy tissue resulting from administration of these MTDs places serious constraints on use of these agents. To balance toxicity with efficacy, conventional dosing schedules call for episodic application of the cytotoxic agent at or close to the MTD, followed by periods of rest to allow normal tissue to recover (Hanahan et al. J. Clinical Invest. 2000 105(8):1045-1047). However, this standard MTD regimen not only seriously impairs the quality of life of the patient, but may also result in only short-lived responses followed by relapses oftentimes by more aggressive cancers resistant to the cytotoxic agent.

Accordingly, alternative therapies are being actively sought. One alternative approach has been to target cells of the vasculature which form the blood vessels of the tumor as opposed to the tumor cells themselves. Angiogenesis is the process of blood vessel formation from pre-existing vasculature and involves recruitment and expansion of the pre-existing endothelium. Angiogenesis is a normally occurring physiological process in the female reproductive cycle and wound healing. However, angiogenesis also occurs in cancer as the establishment of a functional microvasculature is critical for tumor growth and dissemination.

Recent preclinical studies have demonstrated the efficacy of administering the cytotoxic agents cyclophosphamide (Browder et al. Cancer Res. 2000 60:1878-1886) and vinblastine as well as the non cytotoxic VEGF receptor-2 antibody (Klement et al. J. Clinical Invest. 2000 105(8):R15-R24) at shorter intervals without interruption up to 210 days of therapy.

Browder et al. describe administration of cyclophosphamide to mice harboring drug-resistant Lewis Lung carcinoma either daily or every 3,4,5,6,7 or 8 days. In these experiments it was found that cyclophosphamide (170 mg/kg) administered every 6 days was more effective in controlling tumor growth than other cyclophosphamide schedules tested including schedules with a higher dose intensity such as 135 mg/kg every 4 days (Browder et al. Cancer Res. 2000 60:1878-1886).

Klement et al. subjected xenografts of 2 independent neuroblastoma cell lines to either continuous treatment' with low doses of vinblastine, a monoclonal neutralizing antibody (DC101) targeting the flk-1/KDR (type 2) receptor for VEGF, or both agents together. In these experiments, vinblastine was administered at approximately 1.5 mg/m² every 3 days, a dose which is approximately 1/4 of the MTD of this drug in humans and 1/16 to 1/20 of the. MTD in mice (Klement et al. J. Clinical Invest. 2000 105(8):R15-R24).

WO 00/64436 discloses a method for treating infirmities in a subject via administration of a pharmacologically active agent at a sub-therapeutic dose level over an administration period sufficient to achieve a therapeutic benefit. However, no data are provided concerning efficacy of this method for any of the 42 classes of pharmaceutically active agents listed at pages 10-16 of this application is provided.

The administration of drugs at doses lower than the maximum tolerated dose either continuously or at shorter intervals without interruption is oftentimes referred to as chronic or "metronomic" dosing (Hanahan et al. J. Clinical Invest. 2000 105(8):1045-1047).

### Summary of the Invention

An object of the present invention is to provide a metronomic dosing regime for taxanes.

Another object of the present invention is to provide a method for inhibiting tumor growth which comprises exposing the tumor to a taxane via a metronomic dosing regime. This metronomic dosing regime may be used alone or in combination with other established anticancer therapies.

### Detailed Description of the Invention

Tubulin polymerization is generally accepted as one of the most effective targets for cancer chemotherapy. Clinical success in a broad range of cancers has been demonstrated for both commercially available taxanes, TAXOL (paclitaxel) and TAXOTERE (docitaxel). Efficacy of these drugs is schedule dependent with benefits being shown from prolonged tumor exposure times. For example, clinical utility was recently demonstrated using repetitive once weekly administrations of TAXOL.

In addition, preclinical reports indicate that TAXOL may have potent anti-angiogenic activity (Dordunoo et al. Cancer Chemother. Pharmacol. 1995 36:279-82; Burt et al. Cancer Letters 1995 88:73-9; Oktaba et al. Proc. Annu. Meet. Am. Assoc. Cancer Res. 1995 36:A2597; Belotti et al. Proc. Annu. Meet. Am. Assoc. Cancer Res. 1996 37:A397; Belotti et al. Clinical Cancer Res. 1996 2:1843-9; Klauber et al. Cancer research 1997 57:81-6; and Velasco et al. J. invest. Dermatol. 1999 112:655). Since the target population of an anti-angiogenic compound is the endothelium rather than tumor, it has been suggested that to be effective the anti-angiogenic agent must be administered chronically. Unfortunately, the oral bioavailability of commercially available taxanes is very low (<1% in the rat) making chronic repetitive dosing extremely burdensome.

3'-*tert*-Butyl-3'-N-*tert*-butyloxycarbonyl-4-deacetyl-3'-dephenyl-3'-N-debenzoyl-4-O-methoxycarbonyl-paclitaxel is an orally active analog of paclitaxel. The structure of 3'-tert-butyl-3'-N-tert-butyloxycarbonyl-4-deacetyl-3'-dephenyl-3'-N-debenzoyl-4-O-methoxycarbonyl-paclitaxel is depicted in Formula I. Accordingly, this orally bioavailable taxane is referred to hereinafter as the orally active taxane of Formula I. 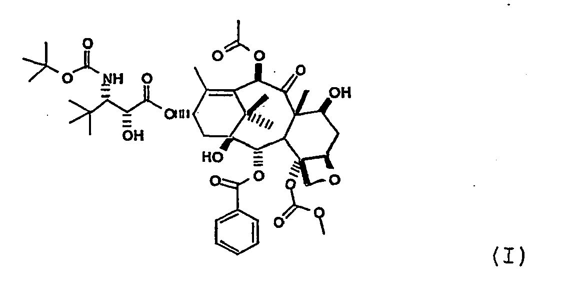 The orally active taxane of Formula I exhibits good oral bioavailability in both the rat and dog, and has anti-tumor activity in multiple human cell lines that is comparable to paclitaxel administered intravenously. An orally effective taxane, referred to as IDN 5109, has also been described (Polizzi et al. Clinical Cancer Res. May 2000 6(5):2070-4; Nicoletti et al. Cancer Res. February 15, 2000 60(4):842-6). In addition, WO99/49848 describes oral formulations of taxanes such as paclitaxel and docetaxel and WO98/53811 describes a dosing regime for taxanes wherein an oral enhancer is also administered.

The present invention relates to a metronomic dosing regime for taxanes, preferably taxanes which are orally bioavailable such as the orally active taxane of Formula I, IDN 5109 or an oral formulation of TAXOL, to inhibit tumor growth and to treat cancer. For purposes of the present invention, by "metronomic dosing regime" it is meant repetitive administration of a drug at a dose below the established maximum tolerated dose for the drug which upon repeated administration produces a desired pharmacological effect with reduced toxic side effects as compared to those observed for the drug when administered at the maximum tolerated dose via a traditional schedule with resting periods. The duration of resting periods may be as great or greater than the duration of treatment which preceded the rest period. In metronomic dosing the same cumulative dose as would be administered via a standard MTD schedule, also referred to herein as induction therapy, may ultimately be administered. In some cases, this is achieved by extending the time frame and/or frequency during which the dosing regime is conducted while decreasing the amount administered at each dose. Thus, by "repetitive" it is meant to be inclusive of chronic and/or continuous dosing regimes. However, the emphasis of metronomic dosing is not so much the frequency nor the duration of therapy so much as it is a relatively safe treatment capable of maintaining the benefit accrued to a patient by the drug when administered via its standard MTD schedule. Accordingly, the taxane administered via the metronomic dosing regime of the present invention is better tolerated by the patient. Metronomic dosing can also be referred to as maintenance dosing or chronic dosing.

For purposes of the present invention, the desired pharmacological effect of metronomic dosing with taxanes is inhibition of tumor growth. "Inhibition of tumor growth" means causing a suppression of tumor growth and/or causing a regression in tumor size. While not being bound to a particular mechanism, it is believed that metronomic dosing with taxanes may target cells of the vasculature which form the blood vessels of the tumor as opposed to the tumor cells themselves. Accordingly, inhibition of tumor growth may result from the inability of the tumor cells to establish the functional microvasculature critical for tumor growth and dissemination.

Toxic side effects reduced by the dosing regime of the present invention include, but are not limited to, neurotoxicity, damage to normal proliferating cells, and weight loss.

Metronomic dosing with an oral taxane may be used alone as a treatment for cancer or in combination or conjunction with other established anticancer therapies administered via standard MTD regimes. Examples of established anticancer therapies which can be used in combination or conjunction with the metronomic dosing regime of the present invention include, but are not limited to, paclitaxel, docetaxel, cyclophosphamide, carboplatin, etoposide, doxorubicin, irinotecan, topotecan, vinblastine, gemcitabine, tegafur/uracil combinations, capecitabine, 5-flurouracil, antibodies such as herceptin, or cetuximab (a.k.a., ERBITUX™) antihormonal treatments such as bicalutamide or flutamide, and radiation therapy. By "combination or in conjunction with" it is meant that the metronomic dosing regime of the present invention is conducted either at the same time as the standard MTD regimen of established anticancer therapies, or more preferably between courses of induction therapy to sustain the benefit accrued to the patient by the induction therapy regimen. When delivered between courses of induction therapy, the intent is to continue to inhibit tumor growth while not unduly compromising the patient's health or the patient's ability to withstand the next course of induction therapy.

The anti-angiogenic activity of the oral taxane of Formula I was evaluated in endothelial cells *in vitro* and in a tumor-independent in vivo angiogenesis model. Both proliferation and tube formation assays were used to evaluate endothelial cell activity *in vitro.*

To assess activity *in vitro,* the effect of the oral taxane of Formula I as compared to TAXOL (paclitaxel) on endothelial cell functions related to the angiogenic process was evaluated. Functions evaluated included proliferation of endothelial cells that form the lumen of the expanding vasculature. As shown in Table 1, the oral taxane of Formula I was nearly equipotent to TAXOL in inhibiting human umbilical vein endothelial cell (HUVEC) proliferation in two separate experiments. Inhibition was also observed for the tumor cell line H3396 at approximately the same concentrations as observed for HUVEC thus indicating that these taxanes exert cytotoxic effects that inhibit proliferation of both endothelial and tumor cells.

**Table 1: Inhibition of HUVEC and H3396 Proliferation**

| **Compound** | **IC**_{**50**} **in Cells [µM]** | | |
|---|---|---|---|
| | **HUVEC** | **H3396** | **H3396/HUVEC** |
| **Formula I** | 0.002, 0.002 | 0.002, 0.003 | 1.25 |
| **TAXOL** | 0.003, 0.005 | 0.003, 0.004 | 0.88 |

Also evaluated were the effects on endothelial cell function that involve differentiation of these cells into tube formation on MATRIGEL. As shown in Table 2, the lowest concentration of these taxanes that resulted in complete inhibition of tube formation on MATRIGEL for both of these taxanes was 0.0500 µM. In addition, further reductions in concentration still retained an inhibitory effect for both of these taxanes.

**Table 2: Inhibition of HUVEC Tube Formation on MATRIGEL**

| Compound | 5.000µM | 0.5000µM | 0.0500µM | 0.0050µM | 0.0005µM |
|---|---|---|---|---|---|
| Formula I | C | C | C | P | P |
| TAXOL | C | C | C | P | P |

| | | | | | |
|---|---|---|---|---|---|
| C= complete; P= partial | | | | | |

Accordingly, these taxanes inhibited two critical processes of angiogenesis, namely endothelial cell proliferation and differentiation. Thus, their anti-tumor effect is not only a consequence of their antiproliferative activity but also a consequence of their activity on other endothelial cell functions.

These taxanes were also evaluated in vivo using MATRIGEL plugs. In these experiments, angiogenic response was measured by evaluating the number of endothelial cells occurring in MATRIGEL plugs at various therapeutic and subtherapeutic doses. The number of endothelial cells occurring in the plugs correlated with the doses tested in a dose-dependent manner for both TAXOL and the oral taxane of Formula I. At the maximum tolerated dose (MTD) of TAXOL, 24 mg/kg, greater than 50% reduction in cell number was observed when compared to control groups on this schedule (every other day for 5 days; q2dx5). For the oral taxane of Formula I, greater than 50% reduction of cell number in plugs was observed at the MTD of 60 mg/kg and at two lower doses of 36 mg/kg and 18 mg/kg. Thus, doses as low as 30% of the MTD still resulted in greater than 50% reduction in cell number. Further, the effect of these taxanes on endothelial cell number, although less at lower doses tested, still resulted in morphological defects as evidenced by the ability of these cells to organize into tubelike structures containing red blood cells when compared to control animals. Thus, anti-angiogenic effects are still observed *in vivo* at doses of the oral taxane of Formula I approximately 13-fold lower than the maximum tolerated dose.

The oral taxane of Formula I was also demonstrated to possess preclinical antitumor efficacy comparable to intravenously administered paclitaxel when administered as a maintenance therapy between courses of induction chemotherapy. In these experiments, mice bearing mammary 16/C murine tumors received either of two general treatment approaches: a) intravenous paclitaxel administered on two consecutive daily treatment schedules separated by an 18 day rest period, i.e. qdx5; 10, 32; or b) intravenous paclitaxel administered on two consecutive daily treatment schedules separated by an 18 day rest period with an additional course of qdx5 therapy consisting of oral administration of the oral taxane of Formula I initiated one week following the end of the first course of intravenous paclitaxel, i.e. paclitaxel qdx5;10,32 + Formula I qdx5; 21. Dose response titrations were performed using each treatment approach. A summary of the gross log cell kill (LCK) values obtained with selected treatment regimens is shown in Table 3.

**Table 3: Effect of Intervening Oral Taxane Maintenance Therapy between Courses of Intravenous Paclitaxel Treatments in Mice Bearing Staged Subcutaneous Mammary 16/C Carcinoma**

| **Treatment (mg/kg/inj)** | | | **Effect** |
|---|---|---|---|
| **Paclitaxel qd 10-14, iv** | **Formula I qd 21-25, po** | **Paclitaxel qd 32-36, iv** | **Gross LCK (cures/total*)** |
| 30 | - | 30 | 10.1 (2/8) |
| 30 | - | 20 | 9.5 |
| 20 | - | 30 | 4.7(1/8) |
| 20 | - | 20 | 4.5 |
| 30 | 20 | 30 | Toxic |
| 30 | 13 | 30 | LD25 |
| 20 | 20 | 20 | >13.8(4/8) |
| 20 | 13 | 20 | 9.0(1/8) |

| | | | |
|---|---|---|---|
| * Cures assessed on Day 88 post-tumor implant. | | | |

Thus, optimal effect obtained with paclitaxel alone, 10.1 LCK including 2 of 8 cures, was obtained at a likely MTD regimen, 30 mg/kg/inj of paclitaxel during each of the two courses of treatment. Lesser amounts of paclitaxel on either or both courses of therapy resulted in diminished efficacy. In comparison, when the oral taxane of Formula I was added to certain intravenous paclitaxel courses of treatment, an improvement in overall efficacy was observed. The optimal combination chemotherapy regimen in these experiments comprised 20 mg/kg/administration of the oral taxane of Formula I in conjunction with 20 mg/kg/injection of intravenous paclitaxel per course of paclitaxel. Further, unlike paclitaxel treatment alone wherein some tumor regrowth occurred during the interval between course therapies, the administration of the oral taxane of Formula I between paclitaxel courses suppressed, and even slightly diminished, the median tumor size of this combination treatment group.

In additional experiments, the oral taxane was offered as maintenance therapy following a single course of induction therapy using intravenous paclitaxel. For mice receiving only intravenous paclitaxel, a MTD regimen consisting of 45 mg/kg/injection, qdx5, iv, beginning on Day 10 post-tumor implant, yielded the same optimum therapeutic outcome as the next lower dose of 30 mg/kg/injection, 1.9 LCK. In contrast, other groups of mice received the induction chemotherapy using paclitaxel, but then received one of two different maintenance regimens using the oral taxane of Formula I. Table 4 provides a summary of the various treatments and outcomes from this experiment.

**Table 4: Effect of Maintenance Therapy with the Oral Taxane of Formula I following Induction Therapy with Intravenous Paclitaxel in Mice Bearing Staged Subcutaneous Mammary 16/C Carcinoma**

| **Treatment (mg/kg/inj)** | | | **Effect** |
|---|---|---|---|
| **Paclitaxel qd 10-14, iv** | **Formula I, po** | | **Gross LCK (cures/total)*** |
| | **q2dx11;d.21** | **q4dx6;d.21** | |
| 45 | - | - | 1.9 (1/8) |
| 30 | - | - | 1.9 (2/8) |
| 45 | 30 | - | Toxic |
| 45 | 13 | - | 3.9 (2/8) |
| 30 | 30 | - | Toxic |
| 30 | 20 | - | Toxic |
| 30 | 13 | - | 3.5 (2/8) |
| 20 | 30 | - | Toxic |
| 20 | 20 | - | 4.0 (1/8) |
| 20 | 13 | - | 2.5 |

| **Paclitaxel qd 10-14, iv** | **Formula I, po** | | **Gross LCK (cures /total)*** |
|---|---|---|---|
| | **q2dx11;d.21** | **q4dx6;d.21** | |
| 45 | - | 45 | LD25 |
| 45 | - | 30 | 5.5 (1/8) |
| 45 | - | 20 | 2.8 |
| 30 | - | 45 | 4.6 (3/8) |
| 30 | - | 30 | 4.4 (2/8) |
| 30 | - | 20 | 3.7 |
| 20 | - | 45 | 2.4 |
| 20 | - | 30 | 2.3 |

| | | | |
|---|---|---|---|
| *Cures assessed on Day 60 post tumor implant. | | | |

The benefits of an extra approximately four weeks of oral taxane are clearly evident in these results. At maximum tolerated combination (paclitaxel + Formula I) regimens, the best LCK achieved was 5.5 with occasional cures as judged at the termination of the experiment (Day 60). The more effective of the oral taxane maintenance therapies did more than prevent tumor progression, they also reduced the tumor burden.

A metronomic dosing regime with a taxane alone was also successful in suppressing growth of human tumor cells in mice. In these experiments, a protracted 30-day treatment schedule using doses of the oral taxane of Formula I below the MTD compared reasonably well with the traditionally used MTD and consolidated schedule approach at suppressing growth of L2987 human lung tumor growth. L2987 human lung tumors were implanted and allowed to reach 50 to 100 mm³ before drug administration. The traditionally used MTD and consolidated schedule approach consisted of a per administration dose of 60 mg/kg delivered orally on the standard schedule (q2dx5). The metronomic dosing regime, while delivering the same cumulative dose of 300 mg/kg, consisted of a per administration dose of 20 mg/kg delivered orally on a modified schedule (every other day for 15 days; q2dx15). While a greater anti-tumor response was observed with the standard schedule, weight loss was also observed. In contrast, the metronomic dosing regime also suppressed tumor growth and no weight loss was observed. Accordingly, metronomic dosing with taxanes provides a safe, yet effective means for inhibiting tumor growth.

As will be understood by those of skill in the art upon reading this disclosure, the metronomic dosing regime used in these experiments merely serves as one example of possible changes in dosing interval and duration which are made to a standard MTD schedule to arrive at an optimal metronomic dosing regime. For example, for the oral taxane of Formula I, metronomic dosing regimes expected to be effective in suppressing tumor growth include, but are not limited to, a daily dosing interval, every other day dosing intervals and dosing once a week. These dosing regimes are extended over periods of time ranging from approximately one month up to at least a year. Drug to be administered in these exemplary metronomic dosing regimes can range from approximately 0.25 mg/M² to 120 mg/M², 0.50 mg/M² to 240 mg/M², and 1 mg/M² to 700 mg/M², respectively. Further, *in vitro* and in vivo angiogenesis experiments provide evidence that cumulative doses lower than 300 mg/kg will also be effective in suppressing tumor growth. Accordingly, metronomic dosing regimes for the oral taxane of Formula I can also be designed for delivery of a lower cumulative dose such as 225 mg/kg, 150 mg/kg, 75 mg/kg, 37.5 mg/kg and even 18.75 mg/kg. Further, metronomic dosing regimes for other taxanes can be routinely designed in accordance with the teachings provided herein for the oral taxane of Formula I based upon their individual standard MTD schedules and their activity in *in vitro* and *in vivo* angiogenesis assays such as those described in the following examples.

The present invention also relates to methods of using metronomic dosing regimes for taxanes to inhibit tumor growth in animals. In a preferred embodiment, the taxane used in these methods will be orally bioavailable. A preferred oral taxane is that of Formula I. However, other taxanes and other means for administering a continuous low dose of the taxane can also be used. For example, other modes of administration of metronomic doses of the present invention include, but are not limited to, via inhalation, intradermally, i.e. via transdermal patches, rectally via suppositories, intramuscularly, intraperitoneally, intravenously and subcutaneously.

For purposes of the present invention, by "animal" it is meant to include any animal in which tumors grow, and in particular humans.

The following nonlimiting examples are provided to further illustrate the present invention.

### EXAMPLES

### Example 1: HUVEC proliferation

Primary human umbilical vein endothelial cells (HUVEC) were purchased from CLONETICS Inc. (San Diego, CA) and used at passage 2 to 3. Proliferation was measured using ³H-thymidine incorporation in cells by pulsing twenty-four hours prior to harvesting cell cultures. The human breast carcinoma line H3396 was used to assess activity of compound on tumor cells. Cells (2 × 10³) were plated on collagen IV coated 96 well plates. Twenty-four hours later, compounds were added at varying concentrations. After 48 hours, ³H-thymidine was added and cells were allowed to incorporate this label over a twenty-four hour period. Cellular extracts were harvested onto glass filters and incorporated radioactivity was determined by counting in a Beta scintillation counter. The IC₅₀, defined as the drug concentration that causes 50% inhibition of ³H-thymidine incorporation, was extrapolated from the plotted data. Cell selective inhibition for endothelial cells by a particular compound was defined by at least a ten-fold greater inhibition of HUVEC primary cell cultures when compared to the H3396 tumor cell line.

### Example 2: In vitro tube formation

Angiogenesis results in a network of functional blood vessels that contain red blood cells. *In vitro* assays that, in part, mimic that process have been established. Primary endothelial cells such as HUVEC when placed on MATRIGEL (Collaborative Research, Inc.) form a three-dimensional network of tubes that align into cords. In this assay system, tube formation was evaluated on an extracellular protein matrix consisting of MATRIGEL diluted 1:1 with culture media (EBM-2; CLONETICS, Inc.) and allowed to polymerize for 60 minutes at 37 C. HUVEC (3.5 × 10⁴)/well in a 24 well plate were distributed in 0.5 ml of media containing vehicle or test compound onto the polymerized MATRIGEL (0.3 ml). Eighteen hours following the plating of cells, the media was removed and cultures were fixed in formalin. Inhibition of tubes on MATRIGEL is evaluated on an inverted microscope using phase contrast lighting.

To determine the effect of compounds in this assay, a descriptive approach was developed. A complete lack of inhibition is defined as when compound exposure at a given concentration results in less than 1% of plated HUVECs occurring as single cells and the remainder of cells form a network or elongated tubelike structure with or without branching. Partial inhibition is defined as an incomplete network with a large number of single cells. Complete inhibition is defined as greater than 99% of cells occurring as single cells with no elongated or branching structures. The number of single cells occurring after vehicle treatment (control) is subtracted from the total number of single cells occurring in the treated groups prior to assessing the effects of test compounds for establishing background effects.

### Example 3: In vivo models and studies

LX1 human lung tumor fragments that were maintained by serial subcutaneous passage in athymic (nu/nu) Balb/c mice were implanted subcutaneously as small fragments approximately 0.1 mm³ in size. Tumor volume doubling time for this tumor in these studies was 2.8 days. When tumors reached the size range of 150-200 mm³, liquid MATRIGEL was injected subcutaneously on the side contralateral to the tumor. Treatment was initiated 24 hours later at varying doses and schedules. Prior to implanting, MATRIGEL is prepared by placing solidified MATRIGEL on ice overnight at 4 C in accordance with the method described by Passiniti et al. (Lab. Invest. 1992 67:519-28). At the liquid phase and while on ice, VEGF and bFGF (Peprotech, Inc. Rocky Hill, NJ) is added to the MATRIGEL at final respective concentrations of 75 ng/ml and 300 ng/ml. Stock solutions of these growth factors are made fresh at 10 mg/ml in PBS. Twenty-four hours following final treatment, animals were sacrificed by cervical dislocation and MATRIGEL plugs from treated and control animals were excised and fixed for at least 48 hours in 10% neutral buffered formalin. These plugs were then processed for paraffin embedding and sectioned at 5 µm thickness followed by staining with hematoxylin and eosin prior to quantitative analysis. The number of endothelial cells in plugs was quantitated using the IMAGEPRO PLUS software (Media Cybernetics, Inc., Silver Spring, MD) at a magnification of 20x. Fifty fields of view from each plug were used for counting the number of endothelial cells. The number of cells were summarized and compared statistically to controls.

Angiogenesis is evaluated in these plugs as the number of endothelial cells migrating into plugs from compound treated groups relative to the vehicle treated group. Tumors were implanted for the purpose of monitoring the anti-tumor effects of these compounds throughout these studies in order to assure compound efficacy at the therapeutic doses.

For dosing, TAXOL .and the oral taxane of Formula I were suspended in 1:1 CREMOPHOR/ethanol solution and delivered at a final concentration of 10% CREMOPHOR and 10% ethanol containing either compound. For TAXOL, normal saline was used as the diluent and delivery was made intravenously. For the oral taxane of Formula I, sterilized water was used as the diluent and delivery was by oral gavage.

### Example 4: Preclinical Studies using Metronomic Dosing in Combination with Established Paclitaxel Therapy

Paclitaxel and the oral taxane of Formula I were dissolved in CREMOPHOR/ethanol (50/50), and then diluted with water (Formula I) or saline (paclitaxel) within approximately one hour of use. The final concentrations of each component of the vehicle was as follows: CREMOPHOR 10%; ethanol 100; aqueous 80%.

C3H conventional mice were purchased from Harlan-Sprague Dawley (Indianapolis, IN) and fed mouse chow and water ad libitum.

The metastatic mammary 16/C murine carcinoma was propagated biweekly in C3H mice. Experiments were initiated by subcutaneous insertion by trocar of tumor fragments.

For *in vivo* tumor testing, the C3H mice were implanted subcutaneously with mammary 16/C tumor fragments. All treatments were initiated on Day 10 post-tumor implant, except for an untreated control group. All groups contained 8 mice. Tumors were measured once or twice weekly and dimensions were converted to weights using the formula Weight (milligrams)= a × b², where a=length and b=width (in millimeters). The median time for tumors within each group of mice to reach 1 gram was determined and the delays in median time to reach 1 gram tumor target size for treated (T) versus control (C) groups was calculated. These delays in tumor growth (T-C value in days) were further converted to gross log cell kill (LCK) values using the formula T-C/(tumor volume doubling time, TVDT, of the control group) x (3.32). A LCK of greater than or equal to 1 LCK was considered an active result. Cures were assessed at the end of each experiment and defined in the absence of a tumor mass of greater than 35 milligrams. Experiments were terminated more than 10 x TVDT following the completion of all treatments.

### Example 5: Phase I Safety, Pharmacokinetic, and Dose Escalation Study of the Oral Taxane of Formula I Administered on a Continuous Daily Metronomic Schedule in Patients with Advance Malignancies

A phase I, open-label, single arm dose escalation study in which cohorts of patients with advanced or metastatic cancer receive escalating doses of the oral taxane of Formula I daily by mouth on an outpatient basis to assess the safety, dose limiting toxicities and optimal bioactive dose of the oral taxane of Formula I has been designed. Pharmacokinetics and pharmacodynamics will also be performed. The study will be conducted on approximately 45 to 65 patients. Starting dose level of the oral taxane will be a fixed dose of 2 mg given once a day on a continuous basis, and on a empty stomach. Doses will be escalated as follows:

| Dose Level | Formula I Dose* | Minimum # of Patients/Cohort |
|---|---|---|
| 1 | 2 mg/day | 6 |
| 2 | 4 mg/day | 6 |
| 3 | 4 mg/m²/day | 6 |
| 4 | 6 mg/m²/day | 6 |
| 5 | 9 mg/m²/day | 6 |
| 6 | 12 mg/m²/day | 6 |

| Dose Level 7 | Formula I Dose* 16 mg/m²/day | Minimum # of Patients/Cohort 6 |
|---|---|---|
| 8 and higher | Increase by increments of 33% of the previous dose | 6 |

All patients will be observed for at least 28 days prior to opening the next dose level for enrollment. Throughout the study, patients may be enrolled in an open dose level simultaneously. Escalation to the next dose level will be permitted if all six patients at the current dose level have completed their first course of treatment and <1 patient has experienced a dose-limiting toxicity during the first course.

Blood samples for pharmacokinetics and pharmacodynamic assessment, as well as surrogate marker evaluation, will be collected from all patients. Plasma markers of endothelial cell activation including sICAM-1, sVCAM-1, sET-1, sE-Selectin and sMCP-1 will be evaluated. Blood and/or tumor samples will also be collected for pharmacogenomics in consenting patients.

To be eligible for the study patients must fulfill all eligibility criteria including, but not limited to, 1) histologically or cytologically confirmed diagnosis of a non-hematologic malignancy which has progressed on standard therapy or for which no standard therapy is known; 2) measurable or non-measurable disease; 3) adequate bone marrow, hepatic and renal function; 4) four weeks elapsed since last dose of immunotherapy, radiotherapy or chemotherapy, including taxanes, (6 weeks for nitrosoureas or mitomycin-C); 5) patients must have recovered to baseline or grade 1 from toxicities resulting from the previous therapies; and 6) Eastern Cooperative Oncology Group performance status 0-1.

Toxicity will be evaluated according to the National Institute of Cancer's Common Toxicity Criteria Version 2.0.

Plasma pharmacokinetics samples of the oral taxane of Formula I will be collected on all patients on Day 1, 8, 15, 22, 29, and 56 and limiting sampling will be obtained every 4 weeks thereafter for patients continuing on the therapy.

### Example 6: Synthesis of the Oral Taxane of Formula I - 3'-tert-Butyl-3'-N-tert-butyloxycarbonyl-4-deacetyl-3'-dephenyl-3'-N-debenzoyl-4-O-methoxycarbonyl-paclitaxel Preparation of (±)-cis-4-tert-Butyl-1-tert-butyloxycarbonyl-3-triethylsilyloxy-azetidin-2-one.

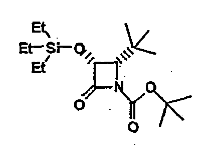

Trimethylacetaldehyde (20.3 mL, 1.25 equiv) was added to a stirred suspension of *p*-anisidine (18.4 gm, 0.150 mole) and anhydrous Na₂SO₄ (150 gm) in anhydrous dichloromethane (250 mL) at room temperature. After 2 hr, this was filtered and the solid was washed with additional anhydrous dichloromethane. The solvent was removed from the filtrate and the crystalline residue was dissolved in anhydrous dichloromethane (750 mL) and placed under a nitrogen atmosphere. Triethylamine (48.0 mL, 2.3 equiv) was added and the reaction was cooled to - 78°C. Benzyloxyacetyl chloride (27.2 mL, 1.15 equiv) was added dropwise and then the reaction was allowed to warm to room temperature. After 24 hr, this was washed with 0.5 M HCl (twice), sat. aqueous NaHCO₃ solution, brine and dried (Na₂SO₄). The solvent was removed and the residue was chromatographed on a silica gel column (gradient elution with 20% dichloromethane in hexane containing 0 to 20% EtOAc) to afford (±)-cis-4-*tert*-butyl-3-benzyloxy-1-*p*-methoxybenzyl-azetidinone as a crystalline solid (46.9 gm, 92%): ¹H NMR (CDCl₃) 1.09 (s, 9H), 3.81 (s, 3H), 4.15 (d, 1H, J=5.5 Hz), 4.77 (d, 1H, J=11.9 Hz), 4.81 (d, 1H, J=5.5 Hz), 5.03 (d, 1H, J=11.9 Hz), 6.87 - 7.43 (m, 9 Hz); LRMS (ESI) 340 ([M+H]⁺). A solution of ceric ammonium nitrate (60.4 gm, 3.6 equiv) im 900 mL of water was added to a well stirred solution of the azetidinone (10.38 gm, 30.6 mmole) in acetonitrile (600 mL) in an ice bath over 1 hr. The reaction was then extracted with EtOAc (twice) and the combined organic extracts were washed with sat. aqueous NaHCO₃ solution (twice), 20% aqueous NaHSO₃ solution, sat. aqueous NaHCO₃ solution and brine. After being dried (Na₂SO₄), the solvents were removed and the residue was chromatographed on a silica gel column (gradient elution with portions of hexane containing 10 to 40% EtOAc) to afford 5.64 gm of slightly impure (±)-cis-3-benzyloxy-4-*tert*-butyl-azetidin-2-one: ¹H NMR (CDCl₃) 1.04 (s, 9H), 3.51 (d, 1H, J=5.2 Hz), 4.71 (m, 2H), 4.96 (d, 1H, J=11.9 Hz), 6.10 (brs, 1H), 7.35 (m, 5H). A suspension of this material (5.54 gm, 23.8 mmole) and 2.5 gm of 10% Pd on charcoal in absolute EtOH (100 mL) was hydrogenated (34 psi H₂, Parr apparatus) for 23 hr. A further 2 gm of the Pd catalyst was added and the hydrogenation was continued for a further 17 hr at 50 psi H₂. The catalyst was removed by filtration and the solvent was removed from the filtrate to leave crude (±)-cis-3-hydroxy-4-(*tert-*butyl)-azetidin-2-one: ¹H NMR (CDCl₃ + 1 drop D₂O) 1.05 (s, 9H), 3.48 (d, 1H, J=5.0 Hz), 4.98 (d, 1H, J=5.0 Hz). This material was dissolved in dry N,N-dimethylformamide (40 mL) and imidazole (3.24 gm, 2 equiv) and triethylsilyl chloride (4.0 mL, 1 equiv) were added. After 10 min, the reaction was partitioned between water and a mixture of EtOAc and hexane (1:1). The organic phase was washed with water (twice), brine and then dried (Na₂SO₄). The solvents were removed and the residue was chromatographed on a silica gel column (gradient elution with 20 to 25% EtOAc in hexane) to give (±)-cis-4-*tert-*butyl-3-triethylsilyloxy-azetidin-2-one (3.86 gm): ¹H NMR (CDCl₃) 0.70 (m, 6H), 0.98 (m, 18H), 3.39 (d, 1H, J=5.0 Hz), 4.88 (dd, 1H, J = 2.1, 5.0 Hz), 6.08 (brs, 1H). A solution of this azetidinone (2.04 gm, 7.92 mmole), diisopropylethyl amine (1.66 mL, 1.2 equiv), di-*tert-*butyl dicarbonate (1.90 gm, 1.1 equiv) and *p-*dimethylaminopyridine (194 mg, 0.2 equiv) in dry dichloromethane (24 mL) was stirred at room temperature for 3 hr. The reaction mixture was diluted with dichloromethane, washed with brine and dried (Na₂SO₄). Removal of the solvent followed by silica gel column chromatography (gradient elution with 0 to 20% EtOAc in hexane) afforded 2.71 gm (96%) of the title compound as an oil: ¹H NMR (CDCl₃) 0.70 (m, 6H), 1.00 (m, 9H), 1.09 (s, 9H), 1.53 (s, 9H), 3.90 (d, 1H, J = 6.5 Hz), 4.93 (d, 1H, J = 6.5 Hz).

### Preparation of baccatin derivative A

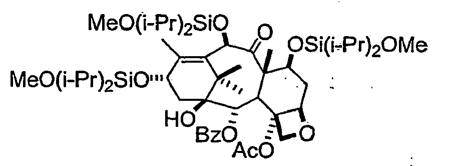

To a solution of 10-desacetylbaccatin (47.4 g, 87 mmol) in anhydrous N,N-dimethylformamide (DMF) (500 mL) was added imidazole (47g, 691 mmol) at ambient temperature. Solution was stirred for 10-15 min until a clear solution was observed. Dropwise, diisopropyldichlorosilane (58 mL, 322 mmol) was added to the reaction mixture. Reaction mixture was stirred for 16 h at ambient temperature. Additional amount of diisopropyldichlorosilane (6 mL) was added to the solution and the reaction mixture was stirred for 60 min. HPLC at this point indicated completion of the reaction. Methanol (36 mL) was added to the mixture and the solution was stirred for 60 min. Reaction was stopped and diluted with a mixture of tert-butyl methyl ketone (TBME) (500 mL) and water (200 mL). Layers were separated and organic phase was washed with brine (250 mL), dried (sodium sulfate) and evaporated to afford the trisilylated baccatin derivative A, (91 g, >100% yield) as a white amorphous compound which was used in the next step without further purification. LRMS(ESI)M+ calcd. For C₅₀H₈₄O₁₃Si₃: 977. Found 977

### Preparation of baccatin derivative B

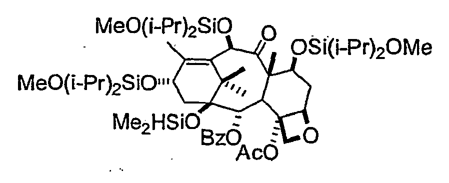

To a solution of baccatin derivative A (90 g, 92 mmol) in DMF (500 mL) was added imidazole (22 g, 320 mmol) at 0°C. Dimethylchlorosilane (35 mL, 320 mmol) was added dropwise at 0 C. Precipitation of the compound was observed at this point. Reaction mixture (slurry) was stirred for 0.5 h at 0°C. Solid was filtered and washed with cold DMF (3X150 mL). After air drying, solid was redissolved in TBME (700 mL) and the solution was washed with water (3 X 200 mL), brine (250 mL) and dried (sodium sulfate). The solution was filtered through a short silica pad. Removal of the solvent under vacuum afforded B in 77% yield (70 g).
LRMS(ESI)M+ calcd. For C₅₀H₉₀O₁₃Si₄: 1035. Found 1035

### Preparation of baccatin derivative C

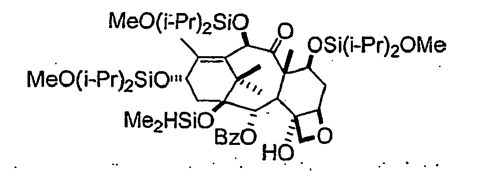

To a stirred solution of B (66.3 g, 64 mmol) in toluene (680 mL) at -34°C was added Red-Al® (50 mL, 160 mmol, 65 wt% solution of sodium bis(2-methoxyethoxy) aluminum hydride in toluene) dropwise over a period of 10 min. Reaction mixture was warmed to -25°C and stirred for 1.5 h. Methanol (62 mL) was added dropwise to the reaction mixture keeping internal temperature between -20 and -25°C. Solution was diluted with TBME ( 500 mL) followed by the addition of 1N sodium hydroxide solution (60 mL) and brine (60 mL). Solution was stirred for 30 min. Diatomaceous earth (12 g) was added to the mixture, stirred for 10 min, and filtered through a pad of diatomaceous earth. Layers were separated. Organic layer was washed with water, brine, and dried (sodium sulfate). Next, solution was passed through a short silica pad before removal of the solvent. The compound was obtained in 97% yield (62 g) as a white solid. LRMS(ESI)M+ calcd. For C₅₀H₈₈O₁₂Si₄: 993. Found 993

### Preparation of baccatin derivative D

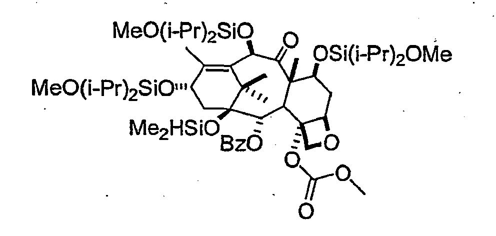

Under argon atmosphere, to a solution of baccatin derivative C (62 g, 62 mmol) in anhydrous tetrahydrofuran (THF) (600 mL) at -60°C was added lithium bis (trimethylsilyl)amide (125 mL, 125 mmol, 1M solution in THF) dropwise. Solution was stirred for 15 min followed by the addition of methyl chloroformate (9 mL, 116 mmol); internal temperature of the solution was maintained at -60°C. Reaction was slowly warmed to 0°C and mixture was stirred for 3 h. After completion of the reaction, saturated ammonium chloride (300 mL) was added. Reaction mixture was extracted with TBME (100 mL). Organic layer was washed with saturated ammonium chloride (200 mL), water (200 mL), brine (200 mL), dried (sodium sulfate), and evaporated to provide D as an oil (67 g, >100%). The crude material was used in the next step without further purification.
LRMS(ESI)M+ calcd. For C₅₂H₉₀O₁₄Si₄: 1051. Found 1051.

### Preparation of baccatin derivative E

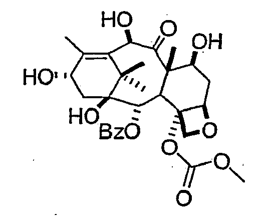

To a solution of baccatin derivative D (62 g, 59 mmol) in dry THF (260 mL) was added triethylamine hydrofluoric acid complex (56 mL, 344 mmol) at ambient temperature. Reaction was stirred for 3 h. Reaction mixture was diluted with ethyl acetate (350 mL) and washed with water (200 mL), brine (200 mL), dried (sodium sulfate), and evaporated to afford E (43 g, >100% crude yield). Reslurring of the crude compound in a mixture of hot ethyl acetate (350 mL) and hexanes (50 mL) gave pure E in 90% yield.
LRMS(ESI)M+ calcd. For C₂₉H₃₆O₁₁: 560. Found 560.

### Preparation of baccatin derivative F

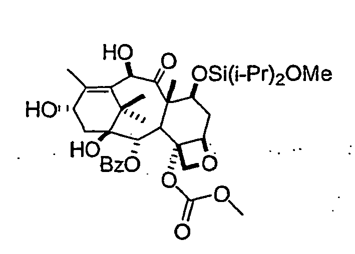

To a stirred solution of baccatin derivative E (32 g, 57 mmol) and imidazole (11.7 g, 172 mmol in DMF (220 mL)) at -65°C was added diisopropyldichlorosilane (26.8 mL) under argon. Temperature of the reaction mixture was maintained at -60°C and the mixture was stirred for 2 h. After completion of the reaction (HPLC), a solution of imidazole in methanol (11.7 g imidazole dissolved in 35 mL methanol) was added and the solution was stirred at 0°C for 30 min. Mixture was extracted with TBME (500 mL). Organic phase was washed with water (4x150 mL), dried (sodium sulfate), and evaporated to afford crude F (45 g). The crude material was further dissolved in acetonitrile (150 mL) and the solution was washed with hexanes (3X100 mL). Removal of acetonitrile afforded pure F as a white solid (34 g, 84% yield).
LRMS(ESI)M+ calcd. For C₃₆H₅₂O₁₂Si: 704. Found 704.

### Preparation of 4-deacetyl-7-[bisisopropyl (methoxy)]silyloxy-4-methoxycarbonyl-baccatin

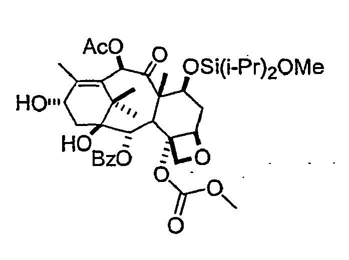

To a solution of baccatin derivative F (33.2 g, 47 mmol) in DMF (200 mL) was added lithium bis (trimethylsilyl)amide (61.2 mL, 61.2 mmol, 1M solution in THF) dropwise at -43°C. The reaction mixture was stirred for 15 min followed by the addition of acetic anhydride (5.8 mL, 63 mmol). The reaction mixture was stirred for 30 min at -40°C. Acetic acid (3.6 mL) was added and the cooling bath was removed. The reaction mixture was extracted with TBME (300 mL) . Organic layer was separated and washed with water (3x150 mL), brine (150 mL), dried (sodium sulfate), and evaporated to afford the crude product. Purification of this compound was achieved by crystallization from a mixture of THF:heptane (1:6). Input of 40 g provided 21 g of crystallized title product (66% yield).
LRMS(ESI)M+ calcd. For C₃₈H₅₄O₁₃Si: 746. Found 746.

### Preparation of 3'-tert-Butyl-3'-N-tert-butyloxycarbonyl-4-deacetyl-3'-dephenyl-3'-N-debenzoyl-4-O-methoxycarbonyl-paclitaxel (Oral Taxane of Formula I)

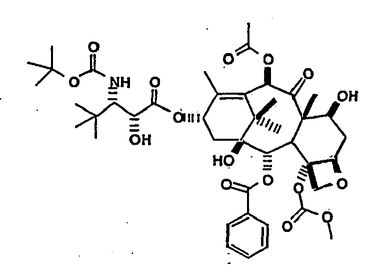

A solution of (+)-cis-4-*tert*-butyl-1-(*tert-*butyloxycarbonyl)-3-triethylsilyloxy-azetidin-2-one (2.71 gm, 5 equiv) and 4-deacetyl-7-[bisisopropyl(methoxy)] silyloxy-4-methoxycarbonyl-baccatin (1.13 gm, 1.52 mmole) in dry THF (100 mL) under N₂ was cooled to -50°C and a solution of lithium bis(trimethylsilyl)amide (1.97 mL, 1.3 equiv, 1.0 M in THF) was added. After 5 min this was transferred to a bath that was maintained at -35 to -30°C for 20 hr and then -25°C for 24 hr. The reaction was then quenched with saturated aqueous NH₄Cl solution and extracted with a mixture of EtOAc and hexane (1:1). The organic extracts were washed with brine and dried (Na₂SO₄). The solvents were removed and the residue was chromatographed (radial chromatography on a 6 mm silica gel plate; gradient elution with 5 to 20% EtOAc in hexane) to afford 1.55 gm of 3'-tert-butyl-3'-N-tert-butyloxycarbonyl-7-[bisisopropyl(methoxy)]silyloxy-4-deacetyl-3'-dephenyl-3'-N-debenzoyl-4-O-methoxycarbonyl-2'-triethylsilyloxy paclitaxel as a mixture of 2', 3'-diastereomers. This mixture was dissolved in dry THF (60 mL) and triethylamine trihydrofluoride (0.92 mL 4 equiv) was added. After 22 hr at room temperature, the reaction mixture was neutralized with saturated aq. NaHCO₃ solution and then extracted with EtOAc. The organic extracts were washed with brine, dried (Na₂SO₄) and the solvents were removed. The residue was chromatographed (radial chromatography; 2 mm silica gel plate; gradient elution from 10 to 50% EtOAc in hexane) to afford (in order of elution): 210 mg (18%) of 2'S,3'R-3'-*tert*-butyl-3-'N-*tert*-butyloxycarbonyl-4-deacetyl-3'-dephenyl-3'-N-debenzoyl-4-O-methoxycarbonyl-paclitaxel {¹H NMR (CDCl₃) 1.04 (s, 9H), 1.13 (s, 3H), 1.20 (s, 3H), 1.37 (s, 9H), 1.65 (s, 1H), 1.66 (s, 3H), 1.84 - 1.93 (m, 2H), 2.17 (s, 3H), 2.25 (s, 3H), 2,55 (m, 3H), 3.00 (d, 1H, J = 6.5 Hz), 3.74 (d, 1H, J = 10.8 Hz), 3.79 (d, 1H, J = 6.9 Hz), 3.92 (s, 3H), 4.16 (d, 1H, J = 8.5 Hz), 4.33 (d, 1H, J = 8.5 Hz), 4.42 (m, 1H), 4.54 (d, 1H, J = 6.5 Hz) 4.87 (d, 1H, J = 10.6 Hz), 5.01 (d, 1H, J = 7.7 Hz), 5.68 (d, 1H, J = 7.0 Hz), 5.76 (m, 1H), 6.32 (s, 1H), 7.44 - 8.05 (m, 5H) ; LRMS (ESI) 846 [(M+H)⁺]} and 668 mg (56%) of the title compound {¹H NMR (CDCl₃) 1.07 (s, 9H), 1.14 (s, 3H), 1.24 (s, 3H), 1.33 (s, 9H), 1.66 (s, 4H), 2.23 (s, 3H), 2.38 - 2.59 (m, 4H), 3.11 (d, 1H, J = 5. 8 Hz), 3.77 (d, 1H, J = 11.1 Hz), 3.82 (d, 1H, J = 7.0 Hz), 3.96 (s, 3H), 4.20 (d, 1H, J = 8.6 Hz), 4.33 (d, 1H, J = 8.6 Hz), 4.39 (m, 1H), 4.53 (d, 1H, J = 5.4 Hz) 4.88 (d, 1H, J = 10.6 Hz), 4.98 (d, 1H, J = 7.9 Hz), 5.69 (d, 1H, J = 7.1 Hz), 6.03 (m, 1H), 6.28 (s, 1H), 7.40 - 8.11 (m, 5H); LRMS (ESI) 846 [(M+H)⁺]}.

## Claims

1. Use of a taxane of Formula I: 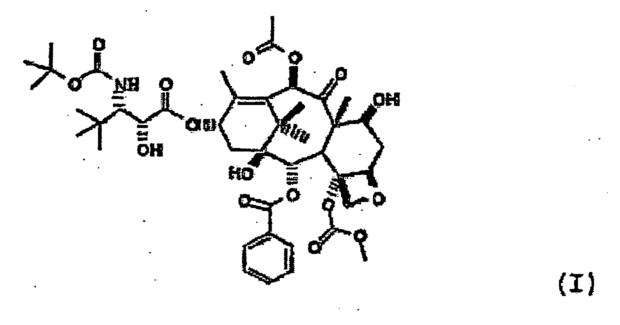 for the manufacture of a medicament for inhibiting tumor growth and producing less toxic side effects, which are neurotoxicity, damage to normal proliferating cells and weight loss, as compared to administration of the maximum tolerated dose of the taxane, wherein the taxane is repeatedly administered at a dose below an established maximum tolerated dose for the taxane.

2. Use of claim 1, wherein the taxane is orally bioavailable.

3. Use of a taxane of Formula I for the manufacture of a medicament for inhibiting growth of tumor cells, wherein the tumor cells are exposed to the taxane via a metronomic dosing regime.

4. Use of a taxane of Formula I for the manufacture of a medicament for inhibiting tumor growth in man or animal, wherein the taxane is administered to man or animal via a metronomic dosing regime.

5. Use according to claims 3 or 4 , wherein the taxane is orally bioavailable.

6. Use according to claims 1, 3 or 4, wherein the taxane is administered with a daily dosing interval or every other day dosing interval over a period of time ranging from approximately one month up to at least a year.

7. Use according to claims 1, 3 or 4, wherein the cumulative dose of the taxane to be administered via the metronomic dosing regime is in the range of less than 300 mg/kg to 18.75 mg/kg.
